# EUROPEAN PATENT APPLICATION

(11) **EP 1 580 553 A1**
(43) Date of publication of application: **28.09.2005**
(21) Application number: 03775958.6
(22) Date of filing: 28.11.2003
(51) Int. Cl.: G01N 33/53, G01N 33/577, C07K 16/18

(54) **METHOD OF ASSAYING ELASTIN DIGESTION PRODUCT AND ASSAY KIT, METHOD OF DETECTING AORTIC DISSECTION AND DETECTION KIT**

(30) Priority: 29.11.2002 JP 2002347423
(71) Applicant: Eisai Co., Ltd., Tokyo 112-8088 (JP)
(72) Inventor: KATAYAMA, Masahiko, Tsukuba-shi, Ibaraki 305-0041 (JP)
(74) Representative: Bannerman, David Gardner
(86) International application number: PCT/JP2003/015265
(87) International publication number: WO 2004/051273

(57) **Abstract**

An immunoassay method for conveniently and accurately measuring amount of the elastin degradation product in a circulating fluid of a subject, which is useful for detection of aortic dissection, the method comprising immunologically binding a first antibody and a second antibody to the elastin degradation product, wherein the first antibody and the second antibody are each an antibody selected from the group consisting of a monoclonal antibody produced by a hybridoma HASG-30 (FERM BP-08489), hybridoma HASG-2 (FERM BP-08488) and hybridoma HASG-61-1 (FERM BP-08490) and antibodies having specificity and affinity to a human aortic pelastin degradation product comparable to those of any of the foregoing monoclonal antibodies.

## Description

### Technical Field

The present invention relates to a method and kit for measuring an elastin degradation product and a method and kit for detecting aortic dissection.

### Background Art

Aortic dissection (also known as aortic aneurysm) is a disease accompanied by sudden chest or abdominal pain and caused by a tear of a part of aortic intima and bleeding from the tear into the intima, resulting in dissection of blood vessel walls. Although the occurrence site is the aorta in most cases, lesions may be extended to vessel branches. As the causes for the onset, aortic dilation and hypertension are also pointed out in addition to genetic factors and denature and weakness of aortic intima (The Japanese Journal of Surgery, 97, 873-878 (1996)). This disease is an acute disease that suddenly occurs without any subjective symptom experienced by patients in advance, and when rupture of the vessel occurs, the fatality rate is very high. Upon onset of the disease, it is standard to immediately conduct surgical procedures for replacing the dissection site in a lesion with an artificial blood vessel. Further, aortic dissection of chest may be accompanied by severe chest pain at the time of onset of the dissection. This is quite similar to the initial symptom of acute myocardial infarction. Acute myocardial infarction can be relatively easily diagnosed by, for example, electrocardiogram, biochemical tests of blood markers etc. However, pathological conditions of aortic dissection cannot be ascertained by these electrocardiogram, biochemical tests of blood markers etc., and also the fatality rate is very high after the onset. Therefore, differential diagnosis of the disease is crucial to selection of therapies (Sogo Rinsho (General Clinic), 48, 2151-2155 (1999)).

For the diagnosis of aortic dissection, it is standard to perform direct visual observation of a lesion in patients by using already established imaging test techniques utilizing special apparatuses such as radiography, CT/MRI angiography and trans-esophageal echogram. The diagnosis is also made by numerically reading the diameter of a dissection site in the blood vessel in images obtained by the foregoing techniques. Further, a diagnostic method is also attempted by periodically performing the aforementioned imaging diagnosis two or more times, calculating an annual rate of expansion of the dissection site from the size at the time of the initial diagnosis as a numerical value and comparing the numerical value with statistical data.

However, all the aforementioned imaging diagnosis techniques require special apparatuses as well as highly sophisticated medical techniques, and patients often have to visit a medical institution specialized for cardiovascular diseases even for diagnosis. Further, a certain degree of physical strain is imposed on patients such as administration of a contrast medium, and considerable time and costs are required to examine only one patient. Furthermore, because these imaging test methods require much time and labor, they are often used for the follow-up of patients having a history of hypertension, arteriosclerosis or the like or patients who already experienced aortic dissection once or more times, and these tests are scarcely performed for patients without a history or subjective symptoms. Further, these methods cannot fully serve as an emergency test for patients who suddenly have had dissection. Therefore, it is desired to establish a technique that enables a convenient, quick and accurate diagnosis, and thus can also be used as an emergency test.

Recently, relations between blood concentration of an elastin degradation product and progress of abdominal aortic dissection has been reported in patients with abdominal aortic dissection (refer to Eur. J. Vasc. Endovasc. Surg., 14, 12-16 (1997)). In this report, it is described that annual expansion rates of aortic aneurysm lesions of patients obtained from imaging tests and numerical values of the abdominal aortic aneurysm lesion sizes in diameter at the initial hospital visit (initial abdominal aortic aneurysms sizes) showed slight positive correlation to blood concentration of the elastin degradation product. Further, more recently, the relationship between urinary concentration of the elastin degradation product and aortic aneurysm diagnosis has been reported (refer to Biol. Pharm. Bull., 22, 854-857(1999)). This report describes that urinary concentrations of the elastin degradation product are statistically different between normal subjects and patients with aortic aneurysm. However, there is no report to study the relationship between the levels of elastin degradation product in a circulating fluid such as blood and the presence or absence of aortic dissection.

It has been shown that the amount of elastin degradation product circulating in human blood can be measured by an immunoassay (refer to Meth. Enzymol., 163, 656-673 (1988); Clin. Physiol. Biochem., 8, 273-282 (1990); J. Immunol. Methods, 164, 175-187 (1993); Eur. J. Vasc. Endovasc. Surg., 14, 12-16 (1997)). However, these immunoassays for measuring the elastin degradation product use polyclonal antibodies derived from antisera obtained by immunizing animals such as rabbits with the elastin degradation product or are based on a competitive technique even when monoclonal antibodies are used, and therefore measurement performances thereof such as specificity and reproducibility are yet to be improved.

### Disclosure of the Invention

A first object of the present invention is to provide an immunoassay method for conveniently and accurately measuring amount of the elastin degradation product in a circulating fluid of a subject, which is useful for detection of aortic dissection, and a kit therefor.

A second object of the present invention is to provide a method for detecting aortic dissection, which enables convenient and accurate detection in a short time and a kit therefor.

To achieve the foregoing objects, the inventors of the present invention prepared multiple kinds of monoclonal antibodies specifically reacting with elastin degradation product derived from human aorta. Then, they resultantly found the useful detection of aortic dissection could be obtained if the elastin degradation product in a human circulating fluid was measured by using a particular combination of antibodies among the prepared antibodies, and thus accomplished the present invention.

The present invention provides an immunoassay method for measuring an elastin degradation product comprising immunologically binding a first antibody and a second antibody to the elastin degradation product, wherein the first antibody and the second antibody are each an antibody selected from the group consisting of a monoclonal antibody produced by a hybridoma HASG-30 (FERM BP-08489), hybridoma HASG-2 (FERM BP-08488) and hybridoma HASG-61-1 (FERM BP-08490) and antibodies having specificity and affinity to a human aortic elastin degradation product comparable to those of any of the foregoing monoclonal antibodies (hereinafter, also referred to as the "assay method of the present invention").

In the assay method of the present invention, it is preferable to use a combination in which the first antibody is the monoclonal antibody produced by the hybridoma HASG-61-1 (FERM BP-08490) and the second antibody is the monoclonal antibody produced by the hybridoma HASG-2 (FERM BP-08488), a combination in which the first antibody is the monoclonal antibody produced by the hybridoma HASG-30 (FERM BP-08489) and the second antibody is the monoclonal antibody produced by the hybridoma HASG-61-1 (FERM BP-08490), or a combination in which each of the first antibody and the second antibody is the monoclonal antibody produced by the hybridoma HASG-2 (FERM BP-08488).

The present invention also provides a method for detecting aortic dissection, which comprises measuring amount of an elastin degradation product in a circulating fluid by the assay method of the present invention and detecting aortic dissection on the basis of a measured value.

The present invention further provides an assay kit for immunoassay of an elastin degradation product comprising a first antibody and a second antibody, wherein the first antibody and the second antibody are each an antibody selected from the group consisting of a monoclonal antibody produced by the hybridoma HASG-30 (FERM BP-08489), hybridoma HASG-2 (FERM BP-08488) and hybridoma HASG-61-1 (FERM BP-08490) and antibodies having specificity and affinity to a human aortic elastin degradation product comparable to those of any of the foregoing monoclonal antibodies (hereinafter, also referred to as "assay kit of the present invention").

In the assay kit of the present invention, it is preferable to use a combination in which the first antibody is the monoclonal antibody produced by the hybridoma HASG-61-1 (FERM BP-08490) and the second antibody is the monoclonal antibody produced by the hybridoma HASG-2 (FERM BP-08488), a combination in which the first antibody is the monoclonal antibody produced by the hybridoma HASG-30 (FERM BP-08489) and the second antibody is the monoclonal antibody produced by the hybridoma HASG-61-1 (FERM BP-08490), or a combination in which each of the first antibody and the second antibody is the monoclonal antibodies produced by the hybridoma HASG-2 (FERM BP-08488).

The assay kit of the present invention is preferably for the detection of aortic dissection.

The present invention further provides a monoclonal antibody produced by the hybridoma HASG-61-1 (FERM BP-08490).

Further, in the assay method of the present invention, the first antibody may be a monoclonal antibody produced by the hybridoma HASG-30 (FERM BP-08489) or an antibody having specificity and affinity to a human aortic elastin degradation product comparable to those of the foregoing monoclonal antibody, and the second antibody may be a monoclonal antibody produced by the hybridoma HASG-2 (FERM BP-08488) or an antibody having specificity and affinity to a human aortic elastin degradation product comparable to those of the foregoing monoclonal antibody. In this embodiment, it is preferred that the first antibody is the monoclonal antibody produced by the hybridoma HASG-30 (FERM BP-08489), and the second antibody is the monoclonal antibody produced by the hybridoma HASG-2 (FERM BP-08488). There is also provided a method for detecting aortic dissection, which comprises measuring amount of an elastin degradation product in a circulating fluid according to this embodiment of the assay method of the present invention and detecting aortic dissection on the basis of a measured value.

Further, in the assay kit of the present invention, the first antibody may be the monoclonal antibody produced by the hybridoma HASG-30 (FERM BP-08489) or an antibody having specificity and affinity to a human aortic elastin degradation product comparable to those of the foregoing monoclonal antibody, and the second antibody may be the monoclonal antibody produced by the hybridoma HASG-2 (FERM BP-08488) or an antibody having specificity and affinity to a human aortic elastin degradation product comparable to those of the foregoing monoclonal antibody. In this embodiment, it is preferred that either the first antibody or the second antibody is immobilized on a solid phase, and the other antibody is labeled with an enzyme. In this embodiment, it is also preferred that the first antibody is the monoclonal antibody produced by the hybridoma HASG-30 (FERM BP-08489), and the second antibody is the monoclonal antibody produced by the hybridoma HASG-2 (FERM BP-08488). The assay kit of the present invention according to this embodiment is also preferably for the detection of aortic dissection.

Furthermore, the present invention also provides the monoclonal antibody produced by the hybridoma HASG-2 (FERM BP-08488) and the monoclonal antibody produced by the hybridoma HASG-30 (FERM BP-08489).

### Brief Description of the Drawings

Fig. 1 shows standard curves representing relationships between aortic elastin degradation product concentrations in test specimens and absorbance determined by enzyme immunoassay.
Fig. 2 shows distributions of serum concentrations of aortic elastin degradation product in patients with aortic dissection and normal elderly males determined by enzyme immunoassay. Combinations of monoclonal antibodies are shown in the parentheses below the identification of the immunoassay systems.
Fig. 3 shows a standard curve representing relationship between aortic elastin degradation product concentrations in test specimens and luminescence counts determined by electrochemiluminescence immunoassay.
Fig. 4 shows distributions of serum concentrations of aortic elastin degradation product in patients with aortic dissection and normal elderly males determined by electrochemiluminescence immunoassay.
Fig. 5 shows a standard curve representing the relationship between human aortic elastin degradation product concentrations and absorbance.

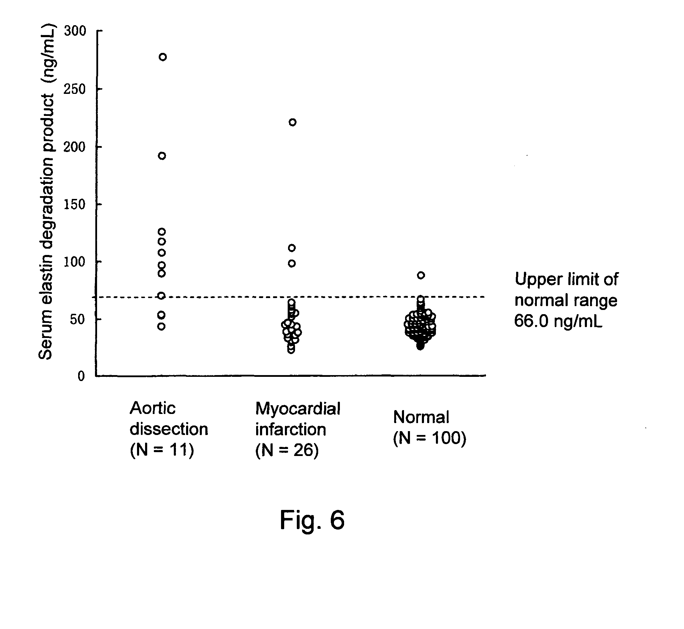

Fig. 6 shows distributions of serum concentrations of elastin degradation product in 11 patients with aortic dissection, 26 patients with myocardial infarction (acute stage) and 100 normal subjects.

### Best Mode for Carrying out the Invention

### <1> Assay method of the present invention

The assay method of the present invention is an immunoassay method for measuring an elastin degradation product comprising immunologically binding a first antibody and a second antibody to the elastin degradation product, wherein the first antibody and the second antibody are each an antibody selected from the group consisting of a monoclonal antibody produced by the hybridoma HASG-30 (FERM BP-08489), hybridoma HASG-2 (FERM BP-08488) and hybridoma HASG-61-1 (FERM BP-08490) and antibodies having specificity and affinity to a human aortic elastin degradation product comparable to those of any of the foregoing monoclonal antibodies.

HASG-2 and HASG-30 were deposited at the International Patent Organism Depositary, National Institute of Advanced Industrial Science and Technology (Postal address: Tsukuba Central 6, 1-1, Higashi 1-Chome, Tsukuba-shi, Ibaraki-ken, 305-8566, Japan) on May 18, 2001 and given accession numbers of FERM P-18335 and FERM P-18336, respectively. Then, the depositions were converted to international depositions under the provisions of the Budapest Treaty on September 18, 2003 and given accession numbers of FERM BP-08488 and FERM BP-08489, respectively. Further, HASG-61-1 was deposited at the International Patent Organism Depositary, National Institute of Advanced Industrial Science and Technology (Postal address: Tsukuba Central 6, 1-1, Higashi 1-Chome, Tsukuba-shi, Ibaraki-ken, 305-8566, Japan) on October 8, 2002 and given an accession number of FERM P-19058. Then, the deposition was converted to an international deposition under the provisions of the Budapest Treaty on September 18, 2003 and given an accession numbers of FERM BP-08490. Antibodies having specificity and affinity to a human aortic elastin degradation product comparable to those of any of the monoclonal antibody produced by HASG-2, monoclonal antibody produced by HASG-30 and monoclonal antibody produced by HASG-61-1 can be selected by the following methods.

The antibodies having comparable specificity and affinity can be selected by, for example, a competitive assay (Antibodies: A Laboratory Manual, Cold Spring Harbor Laboratory (1988) p.567). Specifically, a human aortic elastin degradation product, that is, an antigenic substance, is dissolved in a physiological buffer at an appropriate concentration and adsorbed on a solid phase of microplates. After a blocking treatment, an enzyme-labeled HASG-2, HASG-30 or HASG-61-1 monoclonal antibody and an antibody to be evaluated are added in the same amounts. The antibodies having comparable specificity and affinity can be selected by confirming the performance of inhibiting the reaction of the enzyme-labeled HASG-2, HASG-30 or HASG-61-1 monoclonal antibody.

Alternatively, the antibodies having comparable specificity and affinity can be selected by, for example, the peptide mapping method. Specifically, a human aortic elastin degradation product, that is, an antigenic substance, is isolated by using a high-performance liquid chromatography apparatus or the like on the basis of the difference in molecular weight, hydrophobicity or the like, and binding of an antibody to various isolated elastin antigen fragments is compared with those of HASG-2, HASG-30 and HASG-61-1 monoclonal antibodies to evaluate specificity and affinity of the antibody. An antibody that reacts with antigen fragments, with which HASG-2, HASG-30 or HASG-61-1 monoclonal antibody reacts, in a comparable degree is determined to have specificity comparable to those of the corresponding monoclonal antibody.

The human aortic elastin degradation product can be obtained by the method described in Biochem. J., 61, 11-21 (1955) or can be obtained as a commercial product.

Preferred examples of antibodies to be used include monoclonal antibodies prepared by immunizing a rodent animal such as mouse, rat or hamster with a human aortic elastin degradation product. Although the animal species is not particularly limited, the Balb/C mouse is most commonly used.

Examples of the combinations of the first antibody and the second antibody include a combination in which the first antibody is the monoclonal antibody produced by the hybridoma HASG-30 (FERM BP-08489) or an antibody having specificity and affinity to a human aortic elastin degradation product comparable to those of the foregoing monoclonal antibody and the second antibody is the monoclonal antibody produced by the hybridoma HASG-2 (FERM BP-08488) or an antibody having specificity and affinity to a human aortic elastin degradation product comparable to those of the foregoing monoclonal antibody, and other combinations. Examples of the combination of the first antibody and the second antibody further include a combination in which the first antibody is the monoclonal antibody produced by the hybridoma HASG-30 (FERM BP-08489) and the second antibody is the monoclonal antibody produced by the hybridoma HASG-2 (FERM BP-08488), and other combinations.

The first antibody and the second antibody may be the same antibody. In the assay method of the present invention, it is preferable to use a combination in which the first antibody is the monoclonal antibody produced by the hybridoma HASG-61-1 (FERM BP-08490) and the second antibody is the monoclonal antibody produced by the hybridoma HASG-2 (FERM BP-08488), a combination in the first antibody is the monoclonal antibody produced by the hybridoma HASG-30 (FERM BP-08489) and the second antibody is the monoclonal antibody produced by the hybridoma HASG-61-1 (FERM BP-08490), or a combination in which each of the first antibody and the second antibody is the monoclonal antibody produced by the hybridoma HASG-2 (FERM BP-08488).

The antibodies may be fragments such as Fab, Fab' and F(ab')₂, and may be modified by labeling, immobilization on a solid phase or the like so long as they have required characteristics and affinity.

The assay method of the present invention may be implemented in the same manner as a usual sandwich technique for immunologically binding two kinds of antibodies to an antigen except that a particular combination of antibodies is used.

The immunoassay method is classified into a method of using a radioisotope-labeled compound, a latex agglutination method, a method of using a fluorescence labeled compound, a method of using electrochemiluminescence, a method of using an enzyme and so forth on the basis of the detection method. Although the immunoassay method of the assay method of the present invention is not particularly limited, the assay method of the present invention is preferably an enzyme-linked immunosorbent assay (ELISA) or an electrochemiluminescence immunoassay, because they are safe and convenient.

For ELISA, various methods are known as measurement techniques. In particular, as a convenient method of high quantification ability, a sandwich technique using peroxidase as a labeling enzyme is preferably used. Specifically, either the first antibody or the second antibody is adsorbed on a solid phase such as a bottom surface of a commercially available 96-well microplate. Then, to minimize spontaneous adsorption on the solid phase, a blocking protein such as milk casein is adsorbed. A reference human aortic elastin degradation product solution having a known concentration and a biological test sample are added to the wells and left standing for a predetermined period. After the elastin degradation product antigen in the sample is immunologically bound to the antibody on the solid phase, the plate is washed, and then the other antibody labeled with an enzyme such as peroxidase is added at an appropriate concentration. The reaction mixture is left standing for a predetermined period to form a complex of the three of substances, the antibody on the solid phase, the elastin degradation product antigen and the labeled antibody. Then, the solid phase is washed, and a mixed solution of hydrogen peroxide and a color developing substrate such as ABTS is added to obtain color development by the action of the labeling enzyme. An inhibitor is added to terminate the enzymatic reaction, and then degree of color development is measured in terms of absorbance of the reaction mixture using certain equipment such as a plate reader. The amount of elastin degradation product in a sample solution can be precisely determined by comparing absorbance of the sample solution and absorbance of the reference solution by using a standard curve or the like.

For the electrochemiluminescence immunoassay, various techniques are known as measurement methods. However, as a particularly convenient technique of high quantification ability, a sandwich technique using an antibody immobilized on magnetic beads as the first antibody and an antibody labeled with a chemiluminescent complex, preferably a ruthenium complex, as the second antibody is preferred. In this technique, it is desirable that the first antibody and the second antibody specifically react with an elastin degradation product, and the first antibody and the second antibody may be identical to each other. For the measurement, an anti-elastin degradation product antibody (first antibody) is immobilized on commercially available magnetic beads. Immobilization on the solid phase may be attained with a covalent bond or a noncovalent bond. Then, to minimize non-specific binding of other molecules to the magnetic beads, a blocking protein such as milk casein is adsorbed. An elastin degradation product solution having a known concentration or a biological sample is added to the reaction mixture, and the mixture is stirred for a predetermined period. After the elastin degradation product antigen in the sample is adsorbed on the surfaces of the antibody-bound particles, and then the particles are washed. Then, another anti-elastin degradation product antibody (second antibody) labeled with a chemiluminescent complex, preferably a ruthenium complex, is added at an appropriate concentration. The mixture is stirred for a predetermined period to form a complex of the three substances, the first antibody, the elastin degradation product antigen and the second antibody, on the magnetic beads. Then, the beads are washed, and an electric current is applied between electrodes in a special device to allow luminescence of the complex, which is the labeling compound, of which luminescence intensity is measured. For this luminescence, a luminescence amount corresponding to the amount of the ruthenium-labeled compound can be obtained. The amount of the elastin degradation product in the biological sample can be obtained with good precision by comparing the luminescence amount of the biological sample and luminescence amount of a reference standard using a standard curve or the like.

If the assay method of the present invention is used, patients with aortic dissection show a high blood concentration of elastin degradation product with an extremely high positive rate. While normal subjects scarcely show such a high concentration, an elevated blood concentration is detected in most patients with aortic dissection. Therefore, the assay method of the present invention is effective for diagnosis of the disease.

Accordingly, the present invention also provides a method for detecting aortic dissection, which comprises measuring amount of an elastin degradation product in a circulating fluid by the assay method of the present invention and detecting aortic dissection on the basis of a measured value.

The circulating fluid means any of body fluids circulating in the body such as serum, plasma, cerebrospinal fluid and ascites or fractions thereof, and it is not particularly limited so long as it is a circulating fluid usually collected at a medical institution or the like. However, a fluid derived from blood of a subject such as serum is particularly preferred.

Examples of the method for detecting aortic dissection based on the measured value of elastin degradation product in a circulating fluid include a method of determining the presence of aortic dissection (or high possibility of aortic dissection) when a measured value significantly exceeds the mean value of normal subjects and so forth.

### <2> Assay kit of the present invention

The assay kit of the present invention is an immunoassay kit for measuring an elastin degradation product comprising a first antibody and a second antibody, wherein the first antibody and the second antibody are each selected from the group consisting of a monoclonal antibody produced by the hybridoma HASG-30 (FERM BP-08489), hybridoma HASG-2 (FERM BP-08488) and hybridoma HASG-61-1 (FERM BP-08490) and antibodies having specificity and affinity to a human aortic elastin degradation product comparable to those of any of the foregoing monoclonal antibodies.

The first antibody and the second antibody are as described in the assay method of the present invention.

In the assay kit of the present invention, it is preferred that either the first antibody or the second antibody is immobilized on a solid phase, and the other antibody is labeled with an enzyme or an electrochemiluminescent complex.

Examples of the solid phase include those used in a usual sandwich technique for immunologically binding two kinds of antibodies to an antigen, and the shape and material thereof are not particularly limited. Specific examples include microtiter plates, beads and so forth. Antibodies can be immobilized on a solid phase in a conventional manner.

Examples of the label include those used in a usual sandwich technique for immunologically binding two kinds of antibodies to an antigen, such as radioactive substances, latex, fluorescent substances, chemiluminescent substances, metal colloid particles, enzymes and so forth. In the assay kit of the present invention, the label is preferably an enzyme or an electrochemiluminescent complex. The label and the antibodies can be bound in a conventional manner.

The antibodies contained in the assay kit of the present invention may be in the form of solution or lyophilized product.

The assay kit of the present invention may contain reagents usually used in an immunoassay in addition to the first antibody and the second antibody. Examples of such reagents include a standard antigen (human aortic elastin degradation product) solution, substrate solution, solution for diluting specimen, solution for washing and so forth.

The assay kit of the present invention can be used according to the assay method of the present invention. The assay kit of the present invention is preferably for the detection of aortic dissection.

### Examples

The present invention will be explained more specifically with reference to the following examples. However, the scope of the present invention is not limited to these examples.

### Example 1: Preparation of anti-human aortic elastin degradation product monoclonal antibody and measurement of aortic elastin degradation product in serum by enzyme immunoassay

### 1) Preparation of monoclonal antibody directed to human aortic elastin degradation product

0.1 mg per animal of a human aortic elastin degradation product (Elastin Products Company) was intraperitoneally administered to Balb/C female mice (6-week old) with complete Freund's adjuvant (Difco). After 3 weeks, the same amount of human aortic elastin degradation product was intraperitoneally administered with incomplete Freund's adjuvant (Difco). After further 3 weeks, 0.1 mg per animal of human aortic elastin degradation product alone was administered to the mice. Three days after the final immunization, the spleens were extracted from the mice. The following procedures were performed in a sterile clean bench. The removed spleens were dispersed through a mesh and mixed with Sp2/0-Ag14 mouse myeloma cells cultured beforehand to perform cell fusion in the presence of 50% polyethylene glycol 1500 (Roche Diagnostics). The fused hybridoma cells were dispersed in several 96-well microculture plates and cultured in the RPMI 1640 liquid medium (Sigma-Aldrich) containing 10% fetal calf serum and HAT Reagent (Sigma-Aldrich) for 1 to 2 weeks. During this culture, only hybridoma cells stably producing monoclonal antibodies survived, and unfused myeloma cells or mouse spleen cells died. Cells producing monoclonal antibodies directed to a human aortic elastin degradation product were selected by ELISA using an antigen-immobilized plate. Specifically, when colonies of the hybridoma cells sufficiently grew, the culture supernatant was collected and added to immunogens adsorbed on a 96-well microplate (Nalge Nunc International Corporation) as a solid phase to allow the reaction of the monoclonal antibodies in the supernatant and immunogens. Then, a second antibody reactive to mouse IgG and labeled with peroxidase (Dako Japan) was added at an appropriate concentration. The plate was washed after a predetermined time and an ABTS substrate solution (Roche Diagnostics) was added. Lines of hybridomas producing objective monoclonal antibodies were selected on the basis of the occurrence of color development. The selected lines were subjected to cloning several times. Preparation of the monoclonal antibodies from each line in a large scale was performed in a conventional manner by subjecting mouse ascites to affinity chromatography using protein A-immobilized Sepharose gel (Pharmacia).

### 2) Measurement of aortic elastin degradation product in serum by enzyme immunoassay

The monoclonal antibodies obtained in the above (1) were prepared as purified IgG. The following measurement was performed for various combinations of two kinds of those monoclonal antibodies (combinations of monoclonal antibodies confirmed not to compete each other by a competitive assay). The first monoclonal antibodies were dissolved in PBS at a final concentration of 0.01 mg/mL and a 0.1 mL aliquot was added to each well of a 96-well microplate (Nalge Nunc International Corporation). After adsorption of the contained monoclonal antibodies, the solution was discarded, and 0.2 mL of a PBS solution containing 1% skim milk was added to each well for blocking. After 1 hour, the skim milk solution was discarded, and the wells were washed with Tris-buffered physiological saline containing 0.05% Tween 20 (Sigma-Aldrich) (abbreviated as "Tween-TBS" hereinafter). A reference solution of human aortic elastin degradation product having a predetermined concentration or sample serum was added to the washed well. The sample serum was diluted 10 times with a PBS solution containing 1% skim milk before measurement. The plate was covered with a film and left standing at room temperature for 1 hour, and then, after all the solution was discarded, washed 3 times with Tween-TBS. Then, 0.1 mL of a PBS solution containing 1% skim milk and the second monoclonal antibody labeled with peroxidase (Roche Diagnostics) by the periodic acid method [Antibodies: A Laboratory Manual, by Ed. Harlow & D. Lane, Cold Spring Harbor Laboratory Press p. 348 (1988)] was added to each well. Then, the plate was covered with a film and left standing at room temperature for 1 hour. After the reaction was completed, all the solution on the plate was discarded, and the wells were washed 3 times with Tween-TBS. After washing was completed, 0.1 mL of a TMBZ substrate solution containing hydrogen peroxide (Sigma) was added to each well. The plate was left standing as it was to allow color development for 10 minutes. Then, 0.1 mL of 2 N aqueous hydrochloric acid (Wako Pure Chemical Industries) was added to each well of the plate and mixed well to terminate the reaction. After the reaction was terminated, absorbance of the reaction mixture in each well of the plate was measured at a wavelength of 450 nm by using a microplate reader (Molecular Devices Corporation) to obtain the intensity of the color development as a numerical value.

Examination utilizing the aforementioned assay system revealed that 3 kinds of monoclonal antibodies could be used as materials of the assay system. The hybridomas producing these 3 kinds of monoclonal antibodies were designated as HASG-2, HASG-30 and HASG-61-1. HASG-2 and HASG-30 were deposited at the International Patent Organism Depositary, National Institute of Advanced Industrial Science and Technology (Postal address: Tsukuba Central 6, 1-1, Higashi 1-Chome, Tsukuba-shi, Ibaraki-ken, 305-8566, Japan) on May 18, 2001 and given accession numbers of FERM P-18335 and FERM P-18336, respectively. Then, the depositions were converted to international depositions under the provisions of the Budapest Treaty on September 18, 2003 and given accession numbers of FERM BP-08488 and FERM BP-08489, respectively. HASG-61-1 was deposited at the International Patent Organism Depositary on October 8, 2002 and given an accession number of FERM P-19058. Then, the deposition was converted to an international deposition under the provisions of the Budapest Treaty on September 18, 2003 and given an accession number of FERM BP-08490.

Among the aforementioned enzyme immunoassay systems, the assay system using the monoclonal antibody produced by HASG-30 as the first monoclonal antibody and the monoclonal antibody produced by HASG-2 as the second monoclonal antibody was designated as assay system A. Further, the assay system using the monoclonal antibody produced by HASG-61-1 as the first monoclonal antibody and the monoclonal antibody produced by HASG-2 as the second monoclonal antibody was designated as assay system B. Further, the assay system using the monoclonal antibody produced by HASG-30 as the first monoclonal antibody and the monoclonal antibody produced by HASG-61-1 as the second monoclonal antibody was designated as assay system C.

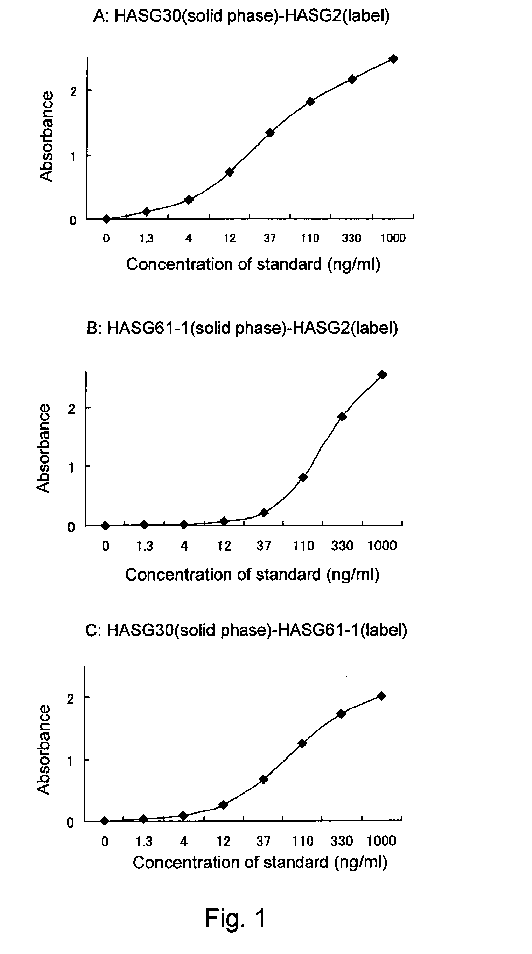

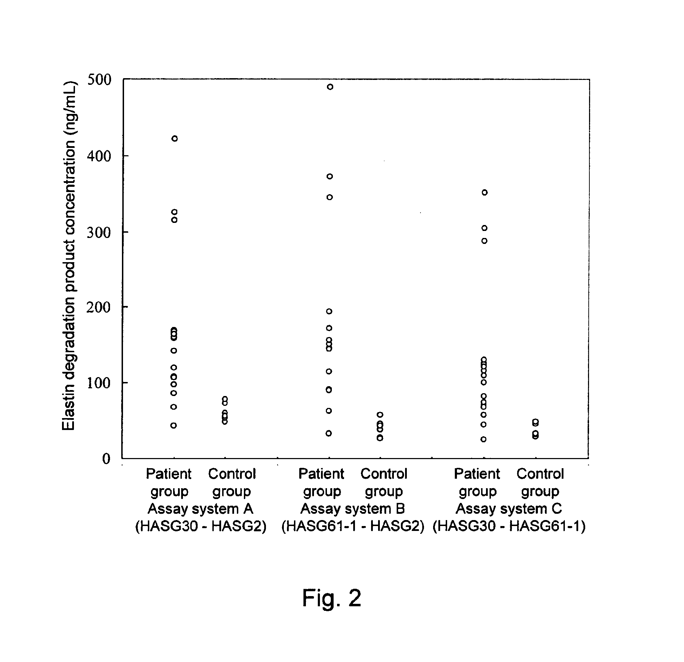

The antigen levels in sera of 17 patients with aortic dissection and 7 subjects of a control group (4 normal male adults, 3 patients with benign disease) were measured by using the aforementioned 3 types of assay systems. Standard curves were created (Fig. 1) from antigen concentrations and absorbance values of the reference human aortic elastin degradation product solutions using a commercially available analysis software program (SOFTmax-J Ver. 2.1, Wako Pure Chemical Industries) to obtain serum antigen concentrations in the test sera. The results are shown in Fig. 2.

The means ± standard deviation of the serum antigen levels of 17 patients with aortic dissection and 7 subjects of the control group obtained by the assay system A were 165.9 ± 96.5 ng/mL and 60.0 ± 10.4 ng/mL, respectively. The means ± standard deviation of the serum antigen levels of 17 patients with aortic dissection and 7 subjects in the control group obtained by the assay system B were 173.4 ± 117.5 ng/mL and 40.0 ± 10.1 ng/mL, respectively. The means ± standard deviation of the serum antigen levels of 17 patients with aortic dissection and 7 subjects in a control group obtained by the assay system C were 128.8 ± 91.5 ng/mL and 35.5 ± 7.6 ng/mL, respectively. The upper limit of the normal range (cutoff value) was tentatively determined for each assay system as a value of the mean serum concentration + two times of the standard deviation of the control group. The upper limits of the normal range were determined to be 80.8 ng/mL for the assay system A, 60.2 ng/mL for the assay system B and 50.7 ng/mL for the assay system C. With the determined upper limits of the normal range, 15 patients out of 17 patients were determined to be positive by the assay system A and the assay system C, and 16 patients out of 17 patients were determined to be positive by the assay system B. Thus, a positive rate of about 90% was obtained for the objective disease in all the assay systems.

### Example 2: Measurement of aortic elastin degradation product in serum by electrochemiluminescence immunoassay

The anti-human aortic elastin degradation product monoclonal antibody produced by HASG-2, which was obtained in Example 1, was prepared as purified IgG and diluted with PBS to a final concentration of 0.2 mg/mL. 0.25 mL of Dynabeads M-450 Epoxyl (Dynal) suspension was added to 1 mL of the antibody solution, sealed in a polypropylene vessel and lightly stirred at room temperature for 4 hours. Then, the mixture was left standing at 4°C for about 12 hours to stabilize the bindings. Then, to block excess binding sites on the bead surfaces, 2 mL of a PBS solution containing 1% skim milk, 0.1% sodium azide, 0.3 mM phenylmethylsulfonyl fluoride (PMSF) and 2 mM EDTA (hereinafter, referred to as SM/PBS) was added to the antibody-bound beads. The mixture was left standing as it was for about 12 hours to stabilize blocking. Then, beads were washed twice with PBS, diluted 20 times with the SM/PBS solution and stored at 4°C as liquid before use.

Further, a solution of purified product of the antibody produced by HASG-2 was separately prepared at a final concentration of 6 mg/mL. 1 mL of this antibody solution, about 2 mg of ruthenium (IGEN, Inc.) was added and the mixture was left standing at room temperature for 2 hours. Then, 2 mL of 0.2 M glycine/PBS (pH 7.8) was added to block excess reaction sites. This ruthenium-labeled antibody solution was subjected to Ultro-gel AcA44 gel chromatography and eluted with PBS containing 0.1% sodium azide, and the labeled antibody eluted first was isolated. The ruthenium-labeled monoclonal antibody of HASG-2 prepared as described above was stored at 4°C as it was before use.

As the standard, a human aortic elastin degradation product (Elastin Products Company, Inc.) was used as in Example 1. Further, the ruthenium-labeled HASG-2 antibody was diluted 100 times with SM/PBS. The measurement of the biological sample was performed by using an automatic electrochemiluminescence immunoassay apparatus (Picolumi 8220, Sanko Junyaku Co., Ltd.) set with a special reaction solution, special washing solution and so forth. A sample serum was diluted 10 times with SM/PBS and used in the measurement. 0.2 mL each of the standard and the diluted sample serum was collected, added to a special reaction tube and set in a special reaction tube rack. The prepared antibody-bound beads and the labeled antibody were also added to the respective special vessels and set in the automatic assay apparatus. The process of the automatic measurement was as follows. First, 0.025 mL of antibody-bound beads were added to the reaction tube, and the reaction with the first antibody was allowed with intermittent stirring for about 3 minutes. The solution in the reaction tube was removed by suction, and then the tube was washed twice with the washing solution. After the washing, 0.2 mL of the ruthenium-labeled antibody solution was added, and the reaction with the second antibody was allowed with intermittent stirring for about 6 minutes. The solution in the reaction tube was removed by suction, and the tube was washed twice with the washing solution. After the washing, 0.3 mL of a luminescent electrolyte solution was added, and the luminescence amount was measured. A standard curve was prepared by using the measured values for the reference antigen simultaneously measured, and the antigen concentration was calculated.

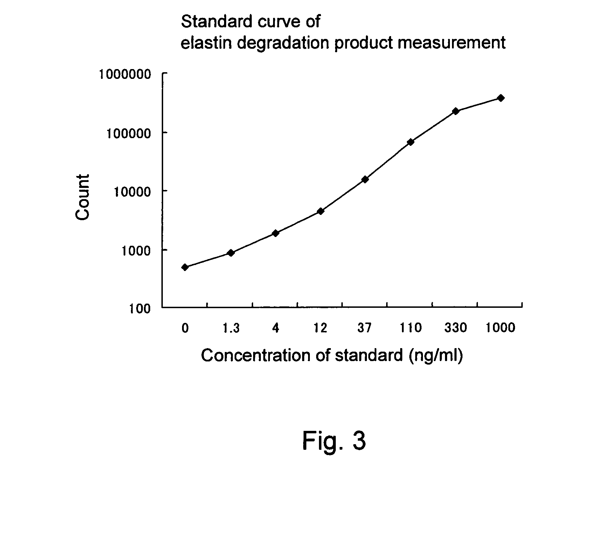

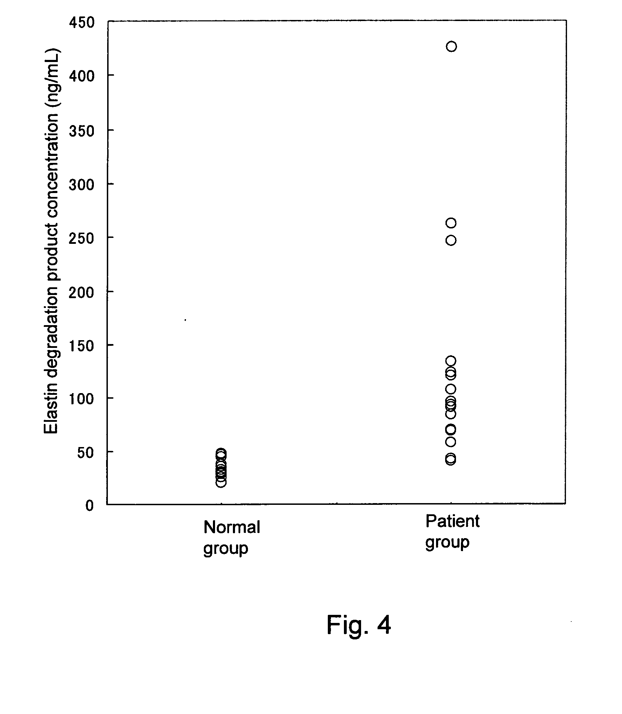

By using the luminescence count obtained with only SM/PBS as a blank value, a standard curve was prepared from antigen concentrations and luminescence counts of the reference human aortic elastin degradation product solutions (Fig. 3), and antigen concentrations in the sample sera were calculated. The results are shown in Fig. 4. The means ± standard deviation of serum antigen levels in 16 patients with aortic dissection and 13 normal elderly males obtained by this measurement were 120.1 ± 101.1 ng/mL and 34.5 ± 8.5 ng/mL, respectively. As the enzyme immunoassay system using two different types of anti-human aortic elastin degradation product monoclonal antibodies described in Example 1, this assay system based on electrochemiluminescence immunoassay also detected aortic dissection with a high positive rate. It was revealed that the measurement performance similar to that of the assay system described in Example 1 could be obtained by a assay system using only 1 kind of HASG-2 antibody as the bead-immobilized antibody and labeled antibody. Further, it was also revealed that considerable reduction of measurement time could be achieved by using electrochemiluminescence immunoassay.

### Example 3: Measurement of aortic elastin degradation product in serum by enzyme immunoassay

The monoclonal antibodies produced by HASG-2 and HASG-30 prepared in Example 1 (1) were prepared as purified IgG. The HASG-2 monoclonal antibody was dissolved in PBS at a final concentration of 0.01 mg/mL, and 0.1 mL of the solution was added to each well of a 96-well microplate (Nalge Nunc International Corporation). After the contained monoclonal antibody was adsorbed, the solution was discarded, and 0.2 mL of a PBS solution containing 1% skim milk was added to each well for blocking. The skim milk solution was discarded 1 hour later, and the wells were washed with PBS. A reference aortic elastin degradation product of a predetermined concentration or a sample serum was added to the washed wells. The plate was covered with a film and left standing at room temperature for 1 hour, then all the solution was discarded, and the wells were washed 3 times with PBS containing 0.05% Tween 20 (Sigma-Aldrich). Then, 0.1 mL of a PBS solution containing 1% skim milk and the HASG-30 monoclonal antibody labeled with peroxidase (Roche Diagnostics) by the periodic acid method [Antibodies: A Laboratory Manual, by Ed. Harlow & D. Lane, Cold Spring Harbor Laboratory Press p.348 (1988)] was added to each well. Then, the plate was covered with a film and left standing at room temperature for 1 hour. After the reaction was completed, all the solution on the plate was discarded, and the wells were washed 3 times with PBS containing 0.05% Tween 20. After the washing was completed, 0.1 mL of an ABTS substrate solution containing hydrogen peroxide (Roche Diagnostics) was added to each well. The plate was left standing as it was to allow color development for 10 minutes. Then, 0.1 mL of 2 mM sodium azide aqueous solution was added to each well of the plate and mixed well to terminate the reaction. After the reaction was terminated, absorbance of the reaction mixture in each well of the plate was measured at a wavelength of 490 nm by using a microplate reader (Molecular Devices Corporation) to obtain the intensity of the color development as a numerical value.

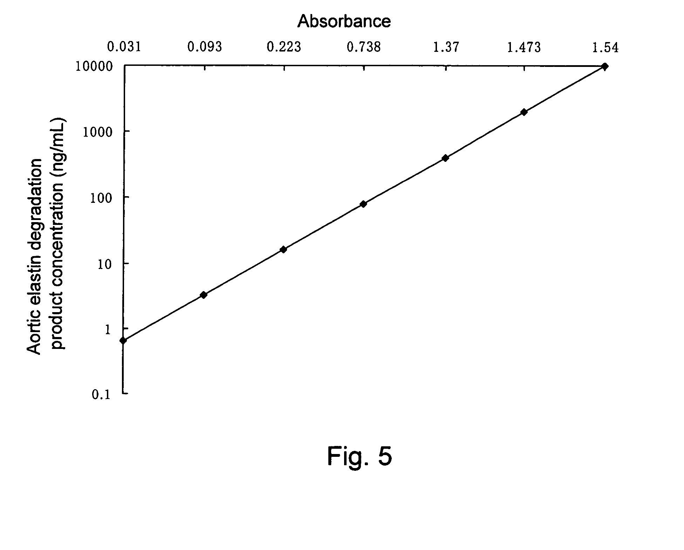

The serum antigen levels in 100 normal adults (70 males, 30 females), 26 patients with acute myocardial infarction and 11 patients with aortic dissection were measured by the aforementioned assay method. A standard curve (Fig. 4) was prepared from antigen concentrations and absorbance values of the reference human aortic elastin degradation product solutions using a commercially available analysis software program (SOFTmax-J Ver.2.1, Wako Pure Chemical Industries), and antigen concentrations in the sample sera were calculated. The results are shown in Fig. 5.

The mean for 100 normal subjects was 44.44 ng/mL. The standard deviation was 10.8 ng/mL. When 66.0 ng/mL was assumed as the upper limit of the normal range as an approximate value of the mean + two times of the standard deviation, 8 patients (72.7%) out of 11 patients with aortic dissection (mean + standard deviation = 111.65 + 66.04 ng/mL), showed positive results, whereas only 3 patients (11.5%) out of 26 patients with acute myocardial infarction (mean + standard deviation = 55.42 + 38.66 ng/mL) showed positive results.

### Industrial Applicability

According to the present invention, aortic dissection can be quickly and conveniently detected with a high positive rate by measuring amount of elastin degradation product in a circulating fluid such as serum without using any special apparatus'.

## Claims

1. An immunoassay method for measuring an elastin degradation product comprising immunologically binding a first antibody and a second antibody to the elastin degradation product, wherein the first antibody and the second antibody are each an antibody selected from the group consisting of a monoclonal antibody produced by a hybridoma HASG-30 (FERM BP-08489), hybridoma HASG-2 (FERM BP-08488) and hybridoma HASG-61-1 (FERM BP-08490) and antibodies having specificity and affinity to a human aortic elastin degradation product comparable to those of any of the foregoing monoclonal antibodies.

2. The method according to claim 1, wherein the first antibody is the monoclonal antibody produced by the hybridoma HASG-61-1 (FERM BP-08490), and the second antibody is the monoclonal antibody produced by the hybridoma HASG-2 (FERM BP-08488).

3. The method according to claim 1, wherein the first antibody is the monoclonal antibody produced by the hybridoma HASG-30 (FERM BP-08489), and the second antibody is the monoclonal antibody produced by the hybridoma HASG-61-1 (FERM BP-08490).

4. The method according to claim 1, wherein each of the first antibody and the second antibody is the monoclonal antibody produced by the hybridoma HASG-2 (FERM BP-08488).

5. A method for detecting aortic dissection, which comprises measuring amount of an elastin degradation product in a circulating fluid by the method according to any one of claims 1 to 4 and detecting aortic dissection on the basis of a measured value.

6. A kit for immunoassay of an elastin degradation product comprising a first antibody and a second antibody, wherein the first antibody and the second antibody are each an antibody selected from the group consisting of a monoclonal antibody produced by the hybridoma HASG-30 (FERM BP-08489), hybridoma HASG-2 (FERM BP-08488) and hybridoma HASG-61-1 (FERM BP-08490) and antibodies having specificity and affinity to a human aortic elastin degradation product comparable to those of any of the foregoing monoclonal antibodies.

7. The kit according to claim 6, wherein the first antibody is the monoclonal antibody produced by the hybridoma HASG-61-1 (FERM BP-08490), and the second antibody is the monoclonal antibody produced by the hybridoma HASG-2 (FERM BP-08488).

8. The kit according to claim 6, wherein the first antibody is the monoclonal antibody produced by the hybridoma HASG-30 (FERM BP-08489), and the second antibody is the monoclonal antibody produced by the hybridoma HASG-61-1 (FERM BP-08490).

9. The kit according to claim 6, wherein each of the first antibody and the second antibody is the monoclonal antibody produced by the hybridoma HASG-2 (FERM BP-08488).

10. The kit according to any one of claims 6 to 9, which is for detection of aortic dissection.

11. A monoclonal antibody produced by a hybridoma HASG-61-1 (FERM BP-08490).

12. The method according to claim 1, wherein the first antibody is the monoclonal antibody produced by the hybridoma HASG-30 (FERM BP-08489) or an antibody having specificity and affinity to a human aortic elastin degradation product comparable to those of the foregoing monoclonal antibody, and the second antibody may be a monoclonal antibody produced by the hybridoma HASG-2 (FERM BP-08488) or an antibody having specificity and affinity to a human aortic elastin degradation product comparable to those of the foregoing monoclonal antibody.

13. The method according to claim 12, wherein the first antibody is the monoclonal antibody produced by the hybridoma HASG-30 (FERM BP-08489), and the second antibody is the monoclonal antibody produced by the hybridoma HASG-2 (FERM BP-08488).

14. A method for detecting aortic dissection, which comprises measuring amount of an elastin degradation product in a circulating fluid by the method according to claim 12 and detecting aortic dissection on the basis of a measured value.

15. The kit according to claim 6, wherein the first antibody is the monoclonal antibody produced by the hybridoma HASG-30 (FERM BP-08489) or an antibody having specificity and affinity to a human aortic elastin degradation product comparable to those of the foregoing monoclonal antibody, and the second antibody is the monoclonal antibody produced by the hybridoma HASG-2 (FERM BP-08488) or an antibody having specificity and affinity to a human aortic elastin degradation product comparable to those of the foregoing monoclonal antibody.

16. The kit according to claim 15, wherein either the first antibody or the second antibody is immobilized on a solid phase, and the other antibody is labeled with an enzyme.

17. The kit according to claim 15, wherein the first antibody is the monoclonal antibody produced by the hybridoma HASG-30 (FERM BP-08489), and the second antibody is the monoclonal antibody produced by the hybridoma HASG-2 (FERM BP-08488).

18. The kit according to claim 15, which is for detection of aortic dissection.

19. A monoclonal antibody produced by a hybridoma HASG-2 (FERM BP-08488).

20. A monoclonal antibody produced by a hybridoma HASG-30 (FERM BP-08489).
